# EUROPEAN PATENT SPECIFICATION

(11) **EP 4 022 304 B1**
(45) Date of publication and mention of the grant of the patent: **02.10.2024**
(21) Application number: 19768984.7
(22) Date of filing: 30.08.2019
(51) Int. Cl.: G01N 33/18

(54) **TOTAL ORGANIC CARBON AND CONDUCTIVITY VERIFICATION AND CALIBRATION USING A SINGLE SAMPLE**
VOLLSTÄNDIGE ORGANISCHE KOHLENSTOFF- UND LEITFÄHIGKEITSÜBERPRÜFUNG UND KALIBRIERUNG ANHAND EINER EINZELPROBE
VÉRIFICATION ET ÉTALONNAGE DE CARBONE ORGANIQUE TOTAL ET DE CONDUCTIVITÉ À L'AIDE D'UN SEUL ÉCHANTILLON

(43) Date of publication of application: 06.07.2022
(73) Proprietor: BL Technologies, Inc., Minnetonka MN 55343 (US)
(72) Inventor: SWANSON, Lukas, Victor, Montana 59875 (US)
(74) Representative: Zimmermann & Partner Patentanwälte mbB
(86) International application number: PCT/US2019/049023
(87) International publication number: WO 2021/040729

(56) References cited:
- WO-A1-2007/053515
- WO-A1-2013/079755
- US-A1- 2007 254 374
- US-B1- 6 228 325

## Description

### BACKGROUND

Total organic carbon (TOC) analyzers and conductivity meters are used for, among other purposes, cleaning validation of pharmaceutical manufacturing systems. TOC is a measurement of the total organic carbon in a sample. Conductivity is a measurement of the conductivity of the sample. TOC analyzers can measure the TOC concentration of a sample, while a separate conductivity meter is used measure the conductivity of the sample.

In order to ensure that a TOC analyzer and a conductivity meter are both working correctly, the accuracy of the TOC analyzer and the conductivity meter may be periodically verified by a technician. Typically, to verify the ability of a TOC analyzer to accurately measure the TOC concentration of a sample, a technician may insert a vial containing a solution having a known TOC value into the TOC analyzer. The technician may then test the TOC of the solution using the TOC analyzer. The technician may then verify that the TOC analyzer is correctly measuring TOC by comparing the results of the test with the known TOC value. The technician may then similarly verify that the conductivity meter is correctly measuring conductivity using a different sample containing a different solution having a known conductivity.

Current methods for verifying TOC analyzers and conductivity meters, while effective, are time consuming. For example, each verification process may require the flushing and/or cleaning of the associated testing equipment, as well as the preparation of a different sample. Accordingly, verifying the TOC of a TOC analyzer and the conductivity of a conductivity meter using different samples is a time-consuming process for technicians.

WO2013079755 discloses a device for predicting the concentration of trihalomethanes in water by measuring the Total Organic Carbon (TOC), the Combined Residual Chlorine (CRC) and the conductivity (C) of said sample.

WO200753515 discloses that it is common practice to periodically calibrate TOC analyzers against one or more known standards which may be acidified.

There are similar issues related to calibrating TOC analyzers and conductivity meters.

### SUMMARY

In an embodiment, a TOC analyzer is provided. The TOC analyzer includes one or more processors and a memory communicably coupled to the one or more processors. The memory stores instructions that when executed by the one or more processors cause the one or more processors to: receive a sample having both a known TOC and conductivity value, wherein the sample comprises an organic acid; measure the TOC concentration of the sample; and measure a conductivity of the sample. The TOC and conductivity are measured at approximately the same time using the same sample resulting in an improvement over existing systems for TOC and conductivity measurement verification.

Embodiments may include some or all of the following features. The instructions may further comprise instructions that cause the one or more processor to verify the TOC and conductivity measuring capability of the TOC analyzer using the measured TOC, the measured conductivity, the known TOC, and the known conductivity of the sample. The organic acid may include citric acid. The organic acid may include lactic acid. The sample may include a single vial containing the organic acid.

In an embodiment, a method for verifying TOC and conductivity measuring capability of a TOC analyzer using a single sample is provided. The method includes: preparing a sample having a known TOC and conductivity, wherein the first sample comprises an organic acid; placing the sample into a TOC analyzer; causing the TOC analyzer to measure a TOC of the sample; and causing the TOC analyzer to measure a conductivity of the sample.

Embodiments may include some or all of the following features. The method may further include: receiving a report from the TOC analyzer, wherein the report comprises results associated with the measurement of the TOC of the sample and the measurement of the conductivity of the sample; and verifying the TOC and conductivity measuring capability of the TOC analyzer using the results and the known TOC and known conductivity of the first sample. The organic acid may include citric acid. The sample may include a single vial containing the organic acid. The TOC and the conductivity may be measured at substantially the same time by the TOC analyzer. The organic acid may include lactic acid.

In an embodiment, a method for calibrating a TOC analyzer with respect to TOC and conductivity is provided. The method includes: receiving a set of samples having a known TOC and conductivity, wherein each sample comprises an organic acid; placing the set of samples into a TOC analyzer; and for each sample of the set of samples: causing the TOC analyzer to measure a TOC of the sample; causing the TOC analyzer to measure a conductivity of the sample; comparing the measured TOC to the known TOC of the sample; comparing the measured conductivity to the known conductivity of the sample; and calibrating the TOC analyzer with respect to TOC and conductivity based on the comparisons.

Embodiments may include some or all of the following features. The method may include receiving a report from the TOC analyzer, wherein the report comprises results associated with the calibration. The organic acid may include citric acid. The sample may include a single vial containing the organic acid. The TOC of the sample and the conductivity of the sample may be measured at approximately the same time. Each sample of the set of samples may have a different known TOC and a different known conductivity. Calibrating the TOC analyzer with respect to TOC and conductivity based on the comparisons may include adjusting one or more offsets based on the comparisons.

### BRIEF DESCRIPTION OF DRAWINGS

Example features and implementations are disclosed in the accompanying drawings. However, the present disclosure is not limited to the precise arrangements and instrumentalities shown.
FIG. 1 is an illustration of an exemplary environment for operating an analyzer.
FIG. 2 is an illustration of an exemplary method for simultaneously verifying TOC and conductivity measurement for an analyzer using a single sample.
FIG. 3 is an illustration of an exemplary method for simultaneously calibrating an analyzer with respect to TOC and conductivity.
FIG. 4 illustrates an example computing device.

### DETAILED DESCRIPTION

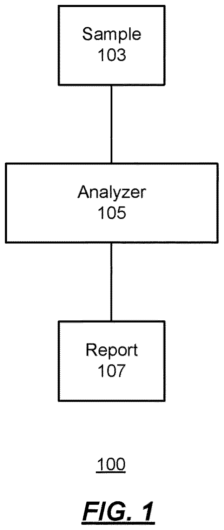

FIG. 1 is an illustration of an environment 100 for verifying and calibrating an analyzer 105. The analyzer 105 may be a TOC analyzer that is adapted to measure the total organic carbon found in a fluid. The analyzer 105 may further be adapted to measure the conductivity of a fluid. The analyzer 105 may include a grab analysis port through which a user may place a sample 103. The sample 103 may include a vial, and the vial may include a fluid or solution. Other types of containers may be used for the sample 103. The fluid may include water or any other fluid or solution that the user desires to measure the TOC or conductivity of.

When the sample 103 is inserted into the grab analysis port of the analyzer 105 the fluid of the sample is tested using one or more needles. The tests that are performed on the sample 103 by the analyzer 105 may be selected by the user through a user interface provided on a display associated with the analyzer 105. For example, the user may select to perform standards such as a conductivity test or a TOC test.

After selecting the desired test, the analyzer 105 may perform the selected test using the fluid of the sample 103. Any method for measuring TOC or conductivity in a fluid may be used. After completing the selected test, the analyzer 105 may generate a report 107 that includes results of the selected test (e.g., the measured TOC and conductivity values). The report 107 may be provided to the user via the display associated with the analyzer 105, via email, or via print. Other methods may be used.

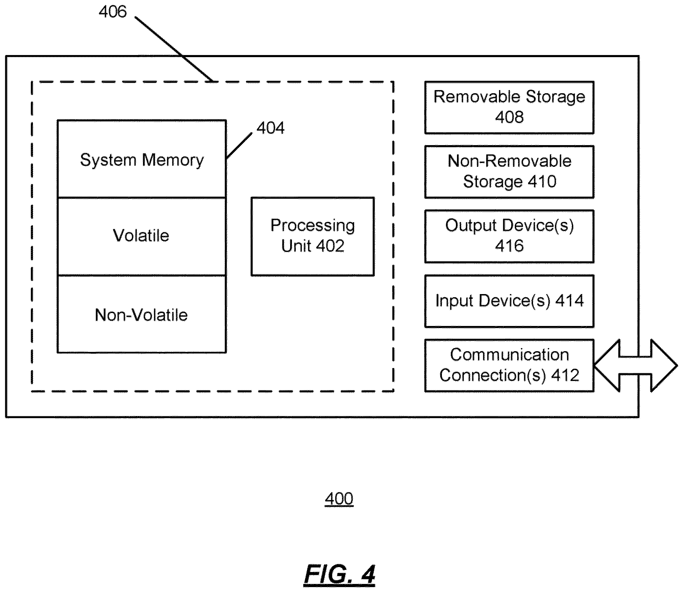

The various functionalities associated with the analyzer 105 (e.g., TOC and conductivity testing, U! display, and report 107 generation) may be implemented using a computing device such as the computing device 400 illustrated with respect to FIG. 4. Depending on the implementation, the computing device may be part of, or separate from, the analyzer 105.

As may be appreciated, to ensure the analyzer 105 is working correctly, the analyzer 105 may provide for both the verification and calibration of the various measurements that are performed by the analyzer 105. Verification may be a single test or measurement that is performed on a fluid with a known TOC value or conductivity value. The results of the test can be compared to the known TOC value and conductivity value to determine if the analyzer 105 is correctly measuring TOC and conductivity of samples 103.

Calibration is the process through which one or more internal values or offsets of the analyzer 105 are adjusted so that a measured TOC or conductivity of a fluid matches a known TOC or conductivity or TOC of the fluid. The calibration process may be performed using multiple steps with each step being performed on a different sample 103 with each sample 103 having a different known TOC and conductivity. The TOC and conductivities measured for each sample 105 may be compared to the known TOC and conductivities for each sample to determine if the analyzer 105 is properly calibrated. One or more offsets of the analyzer 105 may be adjusted based on the comparison.

As described above, currently verification of an analyzer 105 requires the use of two different samples 103 and two different sets of processes or standards. For example, a user or technician may perform a TOC verification process using a sample 103 that includes an inorganic acid such as HCL with a known TOC. After the TOC verification process is completed, the user or technician may then perform a conductivity verification process using a different sample 103 that includes a salt dissolved in a solution having a known conductivity. TOC and conductivity calibration may similarly require the use of two different samples 103 for each step of the calibration. Currently, verifying or calibrating the analyzer 105 may be time consuming task that requires the attention of a technician and prevents the analyzer 105 from being used for a more productive or valuable purpose.

Accordingly, to solve the problems associated with current methods for verifying and calibrating an analyzer 105, a sample 103 is provided that allows both the TOC and conductivity measurement capability of the analyzer 105 to be verified using a single sample 103. The sample 103 further allows for the performing of each step of the calibration process using only a single sample 103.

The sample 103 may include an organic acid solution. The organic acid used may be a triprotic acid that is both organic and conductive. An example acid includes citric acid. However, other acids may be used such as lactic acid or formic acid.

The TOC and conductivity of the sample 103 may be known or set by a user or technician. In some implementations, the organic acid solution used in the sample 103 may be prepared to have a TOC of 500 ppb and a pH of approximately 5. Having a pH of 5 may help stabilize any TOC measurements made on the sample 103 from atmospheric CO₂ contamination, for example.

The sample 103 may include the organic acid in a single vial. A suitable vial is a Dual Use Conductivity and TOC (DUCT) vial. Other types of vials may be used. In some embodiments, the sample 103 may be prepared by a user or technician. Alternatively, the sample 103 may be purchased from a manufacturer for the purposes of verifying or calibrating the analyzer 105 with respect to TOC and conductivity.

When the user or technician desires to verify the analyzer 105 with respect to TOC and conductivity, the user or technician may place the sample 103, including the organic acid solution having a known TOC and conductivity, into the grab analysis port of the analyzer 105. The user or technician may then select a standard or procedure related to verifying both TOC and conductivity using a single sample 103. The standard may be selected using a user interface provided or displayed by the analyzer 105.

The analyzer 105 may then perform both TOC and conductivity verification using the single sample 103 at approximately the same time. Depending on the embodiment, a TOC cell of the analyzer 105 may measure the TOC of the sample 103 as some or all of the organic acid of the sample 103 moves through the analyzer 105. At approximately the same time, or directly after, a conductivity cell of analyzer 105 may measure the conductivity of the sample 103 as the organic acid of the sample 103 continues to move through the analyzer 105. After each measurement, the measured TOC and conductivity may be recorded by the analyzer 105. As may be appreciated, because both the conductivity and the TOC verifications are performed at the same time, the total amount of time required to perform TOC and conductivity verification on an analyzer 105 is effectively halved when compared with previous methods for verifying both TOC and conductivity. Any method for verifying TOC and conductivity by an analyzer 105 may be used.

When the user or technician desires to calibrate the analyzer 105 with respect to TOC and conductivity, the user or technician may receive or prepare a set of samples 103. Each sample 103 may have a different known TOC and a different known conductivity.

As part of the calibration process, the analyzer 105, for each sample 103 of the set of samples 103, may measure the TOC and the conductivity of the sample 103 at approximately the same time as described above for verification. After all of the samples 103 have been measured, the analyzer 105 may compare, for each sample 103, the measured TOC and conductivity with the known TOC and conductivity. If the known TOC and measured TOC for a sample 103 do not match, the analyzer 105 may adjust one or more offsets associated with the TOC cell that measured the TOC of the sample 103. Similarly, If the known conductivity and measured conductivity for a sample 103 do not match, the analyzer 105 may adjust one or more offsets associated with the conductivity cell that measured the conductivity of the sample 103.

Similar to the verification procedure, because both the conductivity and the TOC calibrations procedures are performed at the same time, the total amount of time required to perform TOC and conductivity calibration of an analyzer 105 is also halved. Any method for calibrating an analyzer 105 may be used.

After performing either of the verification or calibration, the analyzer 105 may generate a report 107. The report 107 may include results of one or both of the verification and the calibration. With respect to verification, the results may indicate whether the TOC and conductivity measurements agreed with the known TOC and conductivity of the citric acid solution in the sample 103. With respect to calibration, the report 107 may include results of the calibration including indications of any offsets that were adjusted during the calibration. The report 107 may displayed by the analyzer 105, printed by the analyzer 105, or may be provided electronically by the analyzer 105 (e.g., emailed).

The sample 103 capable of simultaneous verification of the analyzer 105 using a single vile of organic acid described herein provides numerous advantages over prior art systems. First, because the analyzer 105 can be verified (or calibrated) with respect to conductivity and TOC using a single standard or procedure, time and money are saved with respect to manufacturing the sample 103 (e.g., only one sample 103 is made), programming the analyzer 105 to perform the standard or procedure (e.g., only one standard is needed vs. two), and actually performing the verification or calibration (e.g., a technician need only spend half as much time as previously spent). Second, manufacturing a sample 103 with only one vial of a chemical (e.g., citric acid) is cheaper and simpler than manufacturing multiple samples 103 each with a different chemical or combination of chemicals. Third, citric acid may have the additional benefit of promoting a passivation layer within the instruments or cells of the analyzer 105, versus the typically used HCL which removes the passivation layer. The passivation layer may protect the instruments and cells of the analyzer 105 from corrosion.

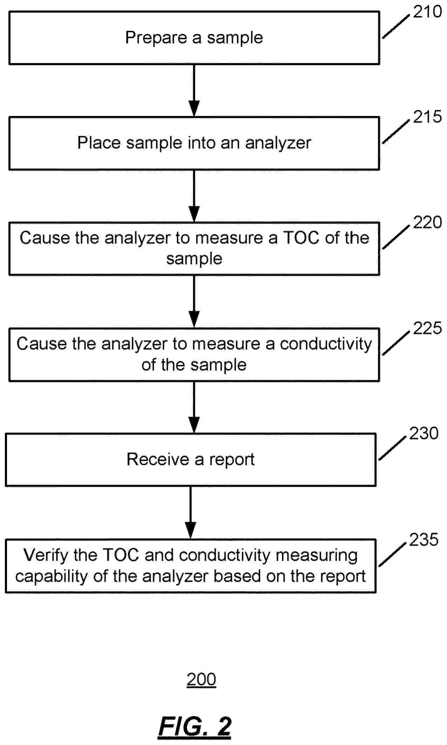

FIG. 2 is an illustration of an exemplary method 200 for verifying TOC and conductivity measurement for an analyzer 105 using a single sample 103. The method 200 may be performed by one or more of a user and an analyzer 105.

At 210, a sample is prepared. The sample 103 may be prepared by a user or technician. The sample 103 may include a vial. The vial may include an organic acid solution such as citric acid. Other organic acids may be used. The organic acid solution may have a known TOC and conductivity.

Alternatively, the user or technician may receive an already prepared sample 103. The sample 103 may be sold for the purposes of verifying or calibrating an analyzer 105 with respect to TOC and conductivity.

At 215, the sample is placed into the analyzer. The sample 103 may be placed into the analyzer 105 by the user or technician. The sample 103 may be placed into the grab analysis port of the analyzer 105.

At 220, the analyzer is caused to measure a TOC of the sample. For example, the user or technician may use an interface of the analyzer 105 to put the analyzer 105 into a verification mode where the analyzer 105 measures the TOC and conductivity of the sample 103. Alternatively, the analyzer 105 may enter the verification mode upon detection of the sample 103 in the grab analysis port. For example, the sample 103 may include a chip, or other indicator, that the analyzer 105 may recognize as being associated with TOC and conductivity verification.

At 225, the analyzer is caused to measure a conductivity of the sample. The analyzer 105 may measure the conductivity of the sample 103 at approximately the same time that it measured the TOC of the sample 103. As described previously, as part of the verification process, the conductivity measurement and the TOC measurement are performed using the same sample 103 at approximately the same time. This is an improvement over prior methods for performing verification which required one sample 103 to verify conductivity and a different sample 103 to verify TOC.

At 230, a report is received. The report 107 may be received by the user or technician that provided the sample 103 and/or started the verification process. The report 107 may be printed or displayed to the user or technician on a display associated with the analyzer 105. The report 107 may include a measured TOC of the sample 103 and a measured conductivity of the sample 103.

At 235, the TOC and conductivity measuring capability of the analyzer is verified. The TOC and conductivity measurement measuring capability of the analyzer 105 may be verified by the user or technician using the known TOC and conductivity values of the sample 103 and the values in the report 107. Alternatively, or additionally, the analyzer 105 may know the expected TOC and conductivity values of the sample 103, and the differences (if any) between the expected and measured values may be indicated in the report 107. Depending on the values in the report 107 the user or technician may recommend performing a calibration of the analyzer 105.

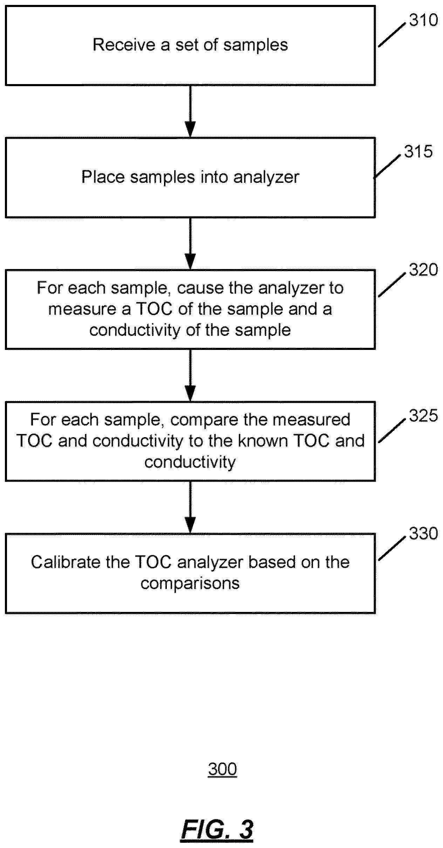

FIG. 3 is an illustration of an exemplary method 300 for calibrating an analyzer 105 with respect to TOC and conductivity. The method 300 may be performed by one or more of a user and an analyzer 105.

At 310, a set of samples is received. The set of samples 103 may be received by a user or technician for purposes of calibrating an analyzer 105 with respect to measuring TOC and conductivity of fluid samples. Each sample 103 may include a vial of an organic acid solution such as a citric acid solution. Each sample may have a different known TOC and a different conductivity.

Alternatively, the user or technician may prepare the samples 103.

At 315, the samples are placed into the analyzer. Each sample 103 may be placed into the grab analysis port of the analyzer 105. In some embodiments, the samples 103 of the set of samples may be placed into the analyzer 105 at the same time. Alternatively, the samples 103 may be placed into the analyzer 105 after each step of the calibration is performed. For example, the analyzer 105 may prompt the user or technician to provide a different sample 103 after each step of the calibration is performed.

At 320, for each sample of the set of set of samples, the analyzer is caused to measure a TOC of the sample and a conductivity of the sample. The TOC and conductivity of each sample 103 may be measured by the analyzer 105 at approximately the same time.

At 325, for each sample of the set of samples, the measured TOC and measured conductivity are compared with the known TOC and the known conductivity of the sample 103. For example, the analyzer 105 may determine the difference (if any) between the measured TOC and the known TOC, and the measured conductivity and the known conductivity. Other methods may be used.

At 330, the analyzer is calibrated based on the comparisons. Depending on the embodiment, the analyzer 105 may be calibrated by adjusting one or more settings, configurations, or offsets of the analyzer 105.

FIG. 4 shows an exemplary computing environment in which example embodiments and aspects may be implemented. The computing device environment is only one example of a suitable computing environment and is not intended to suggest any limitation as to the scope of use or functionality.

Numerous other general purpose or special purpose computing devices environments or configurations may be used. Examples of well-known computing devices, environments, and/or configurations that may be suitable for use include, but are not limited to, personal computers, server computers, handheld or laptop devices, multiprocessor systems, microprocessor-based systems, network personal computers (PCs), minicomputers, mainframe computers, embedded systems, distributed computing environments that include any of the above systems or devices, and the like.

Computer-executable instructions, such as program modules, being executed by a computer may be used. Generally, program modules include routines, programs, objects, components, data structures, etc. that perform particular tasks or implement particular abstract data types. Distributed computing environments may be used where tasks are performed by remote processing devices that are linked through a communications network or other data transmission medium. In a distributed computing environment, program modules and other data may be located in both local and remote computer storage media including memory storage devices.

With reference to FIG. 4, an exemplary system for implementing aspects described herein includes a computing device, such as computing device 400. In its most basic configuration, computing device 400 typically includes at least one processing unit 402 and memory 404. Depending on the exact configuration and type of computing device, memory 404 may be volatile (such as random access memory (RAM)), non-volatile (such as read-only memory (ROM), flash memory, etc.), or some combination of the two. This most basic configuration is illustrated in FIG. 4 by dashed line 406.

Computing device 400 may have additional features/functionality. For example, computing device 400 may include additional storage (removable and/or non-removable) including, but not limited to, magnetic or optical disks or tape. Such additional storage is illustrated in FIG. 4 by removable storage 408 and non-removable storage 410.

Computing device 400 typically includes a variety of computer readable media. Computer readable media can be any available media that can be accessed by the device 400 and includes both volatile and non-volatile media, removable and non-removable media.

Computer storage media include volatile and non-volatile, and removable and non-removable media implemented in any method or technology for storage of information such as computer readable instructions, data structures, program modules or other data. Memory 404, removable storage 408, and non-removable storage 410 are all examples of computer storage media. Computer storage media include, but are not limited to, RAM, ROM, electrically erasable program read-only memory (EEPROM), flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by computing device 400. Any such computer storage media may be part of computing device 400.

Computing device 400 may contain communication connection(s) 412 that allow the device to communicate with other devices. Computing device 400 may also have input device(s) 414 such as a keyboard, mouse, pen, voice input device, touch input device, etc. Output device(s) 416 such as a display, speakers, printer, etc. may also be included. All these devices are well known in the art and need not be discussed at length here.

It should be understood that the various techniques described herein may be implemented in connection with hardware components or software components or, where appropriate, with a combination of both. Illustrative types of hardware components that can be used include Field-programmable Gate Arrays (FPGAs), Application-specific Integrated Circuits (ASICs), Application-specific Standard Products (ASSPs), System-on-a-chip systems (SOCs), Complex Programmable Logic Devices (CPLDs), etc. The methods and apparatus of the presently disclosed subject matter, or certain aspects or portions thereof, may take the form of program code (i.e., instructions) embodied in tangible media, such as floppy diskettes, CD-ROMs, hard drives, or any other machine-readable storage medium where, when the program code is loaded into and executed by a machine, such as a computer, the machine becomes an apparatus for practicing the presently disclosed subject matter.

A number of example implementations are provided herein.

As used in the specification, and in the appended claims, the singular forms "a," "an," "the" include plural referents unless the context clearly dictates otherwise. The term "comprising" and variations thereof as used herein is used synonymously with the term "including" and variations thereof and are open, non-limiting terms. Although the terms "comprising" and "including" have been used herein to describe various implementations, the terms "consisting essentially of" and "consisting of" can be used in place of "comprising" and "including" to provide for more specific implementations and are also disclosed.

## Claims

1. A total organic carbon (TOC) analyzer (105) comprising:
one or more processors (402); and
a memory (404) communicably coupled to the one or more processors and storing instructions that when executed by the one or more processors cause the analyzer to:
receive (215) a sample (103) having a known TOC value and a known conductivity value, wherein the sample comprises an organic acid;
measure (220) a TOC of the sample; and
measure (225) a conductivity of the sample.

2. The TOC analyzer of claim 1, wherein the instructions further comprise instructions that cause the one or more processor to:
verify the TOC and conductivity measuring capability of the TOC analyzer using the measured TOC, measured conductivity, known TOC, and known conductivity of the sample.

3. The TOC analyzer of claim 1, wherein the TOC and the conductivity are measured at substantially the same time.

4. The TOC analyzer of claim 1, wherein the TOC and the conductivity are measured using the same sample.

5. The TOC analyzer of claim 1, wherein the sample comprises a single vial containing the organic acid.

6. A method for calibrating a total organic carbon (TOC) analyzer with respect to TOC and conductivity, comprising:
receiving (310) a set of samples having a known TOC and conductivity, wherein each sample comprises an organic acid;
placing (315) the set of samples into a TOC analyzer; and
for each sample of the set of samples:
causing (320) the TOC analyzer to measure a TOC of the sample;
causing (320) the TOC analyzer to measure a conductivity of the sample;
comparing (325) the measured TOC to the known TOC of the sample;
comparing (325) the measured conductivity to the known conductivity of the sample; and
calibrating (330) the TOC analyzer with respect to TOC and conductivity based on the comparisons.

7. The method of claim 6, wherein the sample comprises a single vial containing the organic acid.

8. The method of claim 6, wherein the TOC of the sample and the conductivity of the sample are measured at approximately the same time.

9. The method of claim 6, wherein each sample of the set of samples has a different known TOC and a different known conductivity.

10. The method of claim 6, wherein calibrating the TOC analyzer with respect to TOC and conductivity based on the comparisons comprises adjusting one or more offsets based on the comparisons.

## Patentansprüche

1. Analysator (105) für den gesamten organischen Kohlenstoff (TOC), umfassend:
einen oder mehrere Prozessoren (402); und
einen Speicher (404), der kommunikativ mit dem einen oder den mehreren Prozessoren gekoppelt ist und Anweisungen speichert, die bei Ausführung durch den einen oder die mehreren Prozessoren den Analysator veranlassen zum:
Empfangen (215) einer Probe (103) mit einem bekannten TOC-Wert und einem bekannten Leitfähigkeitswert,
wobei die Probe eine organische Säure umfasst;
Messen (220) eines TOC der Probe; und
Messen (225) einer Leitfähigkeit der Probe.

2. TOC-Analysator nach Anspruch 1, wobei die Anweisungen ferner Anweisungen umfassen, die den einen oder die mehreren Prozessoren veranlassen zum:
Überprüfen des TOC und der Leitfähigkeitsmessfähigkeit des TOC-Analysators unter Verwendung des gemessenen TOC, der gemessenen Leitfähigkeit, des bekannten TOC und der bekannten Leitfähigkeit der Probe.

3. TOC-Analysator nach Anspruch 1, wobei der TOC und die Leitfähigkeit im Wesentlichen gleichzeitig gemessen werden.

4. TOC-Analysator nach Anspruch 1, wobei der TOC und die Leitfähigkeit unter Verwendung derselben Probe gemessen werden.

5. TOC-Analysator nach Anspruch 1, wobei die Probe aus einem einzelnen Fläschchen besteht, das die organische Säure enthält.

6. Verfahren zum Kalibrieren eines Analysators für den gesamten organischen Kohlenstoff (TOC) in Bezug auf TOC und Leitfähigkeit, umfassend:
Empfangen (310) eines Satzes von Proben mit bekanntem TOC und bekannter Leitfähigkeit, wobei jede Probe eine organische Säure umfasst;
Platzieren (315) des Probensatzes in einen TOC-Analysator; und
für jede Probe des Probensatzes:
Veranlassen (320), dass der TOC-Analysator einen TOC der Probe misst;
Veranlassen (320), dass der TOC-Analysator eine Leitfähigkeit der Probe misst;
Vergleichen (325) des gemessenen TOC mit dem bekannten TOC der Probe;
Vergleichen (325) der gemessenen Leitfähigkeit mit der bekannten Leitfähigkeit der Probe; und
Kalibrieren (330) des TOC-Analysators hinsichtlich des TOC und der Leitfähigkeit basierend auf den Vergleichen.

7. Verfahren nach Anspruch 6, wobei die Probe ein einzelnes Fläschchen umfasst, das die organische Säure enthält.

8. Verfahren nach Anspruch 6, wobei der TOC der Probe und die Leitfähigkeit der Probe ungefähr zur gleichen Zeit gemessen werden.

9. Verfahren nach Anspruch 6, wobei jede Probe des Probensatzes einen unterschiedlichen bekannten TOC und eine unterschiedliche bekannte Leitfähigkeit aufweist.

10. Verfahren nach Anspruch 6, wobei das Kalibrieren des TOC-Analysators in Bezug auf das TOC und der Leitfähigkeit basierend auf den Vergleichen das Anpassen eines oder mehrerer Offsets basierend auf den Vergleichen umfasst.

## Revendications

1. Analyseur de carbone organique total (COT) (105) comprenant :
un ou plusieurs processeurs (402) ; et
une mémoire (404) couplée de manière communicable à un ou plusieurs processeurs et stockant des instructions qui, lorsqu'elles sont exécutées par un ou plusieurs processeurs, amènent l'analyseur à :
recevoir (215) un échantillon (103) ayant une valeur connue de COT et une valeur connue de conductivité,
dans lequel l'échantillon comprend un acide organique ;
mesurer (220) un COT de l'échantillon ; et
mesurer (225) une conductivité de l'échantillon.

2. Analyseur de COT selon la revendication 1, dans lequel les instructions comprennent en outre des instructions qui amènent le ou les processeurs à :
vérifier la capacité de mesure du COT et de la conductivité de l'analyseur de COT à l'aide du COT mesuré, la conductivité mesurée, le COT connu et la conductivité connue de l'échantillon.

3. Analyseur de COT selon la revendication 1, dans lequel le COT et la conductivité sont mesurés pratiquement en même temps.

4. Analyseur de COT selon la revendication 1, dans lequel le COT et la conductivité sont mesurés à l'aide du même échantillon.

5. Analyseur de COT selon la revendication 1, dans lequel l'échantillon comprend un seul flacon contenant l'acide organique.

6. Procédé d'étalonnage d'un analyseur de carbone organique total (COT) par rapport au COT et à la conductivité :
la réception (310) d'un ensemble d'échantillons ayant un COT et une conductivité connus, chaque échantillon comprenant un acide organique ;
le placement (315) de la série d'échantillons dans un analyseur COT ; et
pour chaque échantillon de la série d'échantillons :
le fait d'amener (320) l'analyseur de COT à mesurer le COT de l'échantillon ;
le fait d'amener (320) l'analyseur de COT à mesurer la conductivité de l'échantillon ;
la comparaison (325) du COT mesuré au COT connu de l'échantillon ;
la comparaison (325) de la conductivité mesurée à la conductivité connue de l'échantillon ; et
l'étalonnage (330) de l'analyseur de COT en ce qui concerne le COT et la conductivité sur la base des comparaisons.

7. Procédé selon la revendication 6, dans lequel l'échantillon comprend un seul flacon contenant l'acide organique.

8. Procédé selon la revendication 6, dans lequel le COT de l'échantillon et la conductivité de l'échantillon sont mesurés approximativement au même moment.

9. Procédé selon la revendication 6, dans lequel chaque échantillon de la série d'échantillons a un COT connu différent et une conductivité connue différente.

10. Procédé selon la revendication 6, dans lequel l'étalonnage de l'analyseur de COT par rapport au COT et à la conductivité sur la base des comparaisons comprend l'ajustement d'un ou de plusieurs décalages sur la base des comparaisons.
